# EUROPEAN PATENT APPLICATION

(11) **EP 2 324 784 A1**
(43) Date of publication of application: **25.05.2011**
(21) Application number: 10251986.5
(22) Date of filing: 23.11.2010
(51) Int. Cl.: A61B 17/34

(54) **Surgical portal and introducer assembly**

(30) Priority: 24.11.2009 US 263912 P; 11.10.2010 US 901643
(71) Applicant: Tyco Healthcare Group LP, New Haven, CT 06511 (US)
(72) Inventor: Kleyman, Gennady, Brooklyn, NY 11230 (US)
(74) Representative: Soames, Candida Jane

(57) **Abstract**

A surgical portal and introducer assembly includes an introducer and a portal. The introducer has a surface member and a portal member extending from the surface member. The surface member limits the positioning of the portal member within a tissue tract relative to an outer tissue surface. The portal member and the surface member define a longitudinal channel therethrough. The portal is positionable within the longitudinal channel of the introducer. The portal has one or more longitudinal ports dimensioned to permit passage of a surgical object therethrough. The portal includes a compressible material that permits the portal to transition between a first expanded condition and a second compressed condition. The portal is biased toward the first expanded condition. The portal maintains a substantially scaled relationship with the longitudinal channel when positioned in the longitudinal channel and disposed in the expanded condition.

## Description

### CROSS-REFERENCE TO RELATED APPLICATIONS

This application claims priority to, and the benefit of, U.S. Patent Application Serial Number 61/263,912 filed on November 24, 2009, the entire contents of which are incorporated herein by reference.

### BACKGROUND

### Technical Field

The present disclosure relates generally to surgical portals for use in minimally invasive surgical procedures, such as endoscopic and/or laparoscopic procedures, and more particularly, relates to a surgical portal and an associated introducer to assist in inserting the surgical portal into a tissue tract of a patient.

### Description of Related Art

Today, many surgical procedures are performed through small incisions in the skin, as compared to the larger incisions typically required in traditional procedures, in an effort to reduce both trauma to the patient and recovery time. Generally, such procedures are referred to as "endoscopic", unless performed on the patient's abdomen, in which case the procedure is referred to as "laparoscopic." Throughout the present disclosure, the term "minimally invasive" should be understood to encompass both endoscopic and laparoscopic procedures.

During a typical minimally invasive procedure, surgical objects, such as surgical access devices, e.g., trocar and cannula assemblies, or endoscopes, are inserted into the patient's body through the incision in tissue. In general, prior to the introduction of the surgical object into the patient's body, insufflation gas are used to enlarge the area surrounding the target surgical site to create a larger, more accessible work area. Accordingly, the maintenance of a substantially fluid-tight seal is desirable so as to prevent the escape of the insufflation gases and the deflation or collapse of the enlarged surgical site.

To this end, various ports with valves and seals are used during the course of minimally invasive procedures and are widely known in the art. However, a continuing need exists for an access port and associated introducer, which can position the access port with relative ease and with minor inconvenience for the surgeon.

### SUMMARY

Accordingly, a surgical portal and introducer assembly includes an introducer and a portal. In one embodiment, the surgical portal and introducer assembly includes a spacer that couples with the introducer. The introducer is dimensioned for at least partial positioning within a tissue tract and is generally secured to the tissue tract in substantial sealed relationship with the tissue tract. The introducer has a surface member and a portal member extending from the surface member. The portal member and the surface member define a longitudinal channel therethrough. The introducer may be formed of a substantially rigid material. The rigid material comprises one or both of steel or plastic.

The surface member is dimensioned to limit the positioning of the portal member within a tissue tract relative to an outer tissue surface. The surface member may define a plurality of passages therethrough. One or more of the passages are dimensioned for the reception of a surgical object. One or more of the passages may be disposed in mechanical cooperation with an insufflation conduit for the reception of insufflation fluid. The surface member may include one or more projections extending therefrom. The one or more projections may be actuable between a first position and one or more second positions. In the first position, the one or more projections extend vertically from the surface member and are substantially parallel with a longitudinal axis of the introducer. In the one or more second positions, the one or more projections are disposed at an angle relative to the longitudinal axis of the introducer.

In one embodiment, the introducer may be separable into first and second sections. One or both of the first and second sections includes one or more grooves and one or both of the first and second sections includes one or more pins extending therefrom. The one or more pins and the one or more grooves are engageable to couple the first and second sections.

The portal is positionable within the longitudinal channel of the introducer. The portal has one or more longitudinal ports dimensioned to permit passage of a surgical object therethrough. The portal includes a compressible material that permits the portal to transition between a first expanded condition and a second compressed condition to facilitate passage through the longitudinal channel of the introducer. The portal is biased toward the first expanded condition. The portal maintains a substantially sealed relationship with the longitudinal channel of the introducer when positioned in the longitudinal channel and disposed in the expanded condition. The portal may be formed of a non-rigid material. The non-rigid material comprises one or more of foam, gel material or soft rubber.

The portal defines a plurality of longitudinal ports dimensioned to permit passage of a surgical object therethrough. The portal includes a flange segment on each of the leading and trailing ends such that the portal defines a substantially hour-glass shape. Each of the flange segments has a larger diameter than the diameter of the longitudinal channel of the introducer when the portal is in the expanded condition. The portal is formed of a material having sufficient flexibility to accommodate off-axis motion of a surgical object positioned within the one or more longitudinal ports. The one or more longitudinal ports are substantially parallel to a longitudinal axis of the portal.

According to one aspect, the present disclosure is directed to a method of accessing a surgical site. The method includes the steps of providing an introducer having a surface member and a portal member extending from the surface member, wherein the surface member and the portal member define a longitudinal channel therethrough and providing a portal having two or more longitudinal ports. The method involves the steps of advancing the introducer into tissue to a predetermined depth, compressing the portal to fit within the longitudinal channel of the introducer, advancing the portal at least partially through the longitudinal channel of the introducer, and expanding the portal into sealing engagement with the longitudinal channel of the introducer. The method may include the step of simultaneously advancing two or more surgical objects through the two or more longitudinal ports of the portal. The method may involve actuating the one or more vertical projections in order to remove the introducer from the tissue wherein the introducer includes one or more vertical projections extending from the surface member. According to one step, the method may include advancing the introducer into tissue with an obturator.

### DESCRIPTION OF THE DRAWINGS

The above and other aspects, features, and advantages of the present disclosure will become more apparent in light of the following detailed description when taken in conjunction with the accompanying drawings in which:

FIG. 1 is a perspective view of a surgical portal and introducer assembly including a surgical portal and an introducer in accordance with the principles of the present disclosure;

FIG. 2 is a perspective view of the introducer of FIG. 1 with an obturator positioned therethrough, both the introducer and the obturator being inserted into tissue;

FIG. 3 is perspective view of another embodiment of an introducer and another embodiment of an obturator in accordance with the present disclosure;

FIG. 4 is a perspective view of a first section of the introducer of FIG. 3; and

FIG. 5 is a perspective view of another embodiment of a surgical portal and introducer assembly including the introducer of FIG. 3, the surgical portal of FIG. 1, and a spacer.

### DETAILED DESCRIPTION

Particular embodiments of the present disclosure will be described herein with reference to the accompanying drawings. As shown in the drawings and as described throughout the following description, and as is traditional when referring to relative positioning on an object, the term "proximal" or "trailing" refers to the end of the apparatus that is closer to the user and the term "distal" or "leading" refers to the end of the apparatus that is farther from the user. In the following description, well-known functions or constructions are not described in detail to avoid obscuring the present disclosure in unnecessary detail.

One type of minimal invasive surgery described herein employs a device that facilitates multiple instrument access through a single incision. This is a minimally invasive surgical procedure, which permits a surgeon to operate through a single entry point, typically the patient's navel. The device may also be used in a naturally occurring orifice (e.g., anus or vagina). The disclosed procedure involves insufflating the body cavity and positioning a portal member within, e.g., the navel of the patient. Instruments including an endoscope and additional instruments such as graspers, staplers, forceps or the like may be introduced within the portal member to carry out the surgical procedure. An example of such a surgical portal is disclosed in commonly assigned U.S. patent application Serial No. 12/244,024, filed October 2, 2008, the entire content of which is hereby incorporated by reference herein.

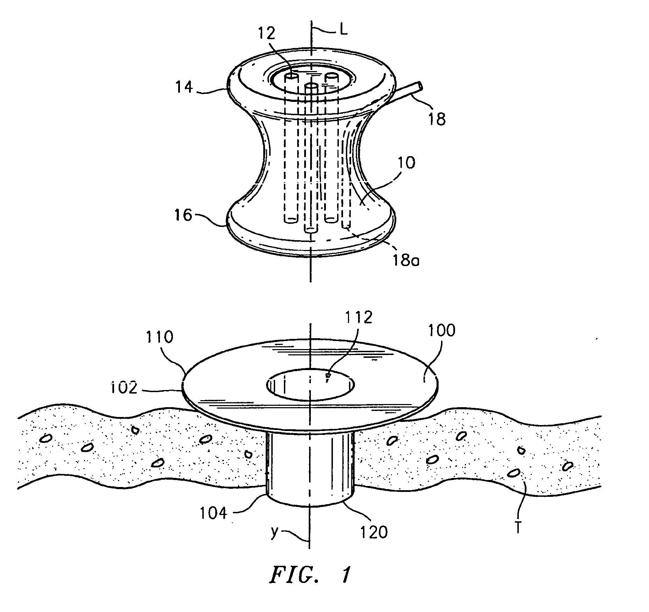

Referring now to the drawings, in which like reference numerals identify identical or substantially similar parts throughout the several views, FIG. 1 illustrates a surgical portal and an introducer assembly including a surgical portal 10 and an introducer 100 in accordance with the principles of the present disclosure.

Surgical portal 10 and introducer 100 are adapted for insertion within a tract defined within tissue "T", e.g., through the abdominal or peritoneal lining in connection with a laparoscopic surgical procedure. In particular, when inserted within the tissue "T", introducer 100 is adapted to establish a substantial seal within the tract defined within the tissue "T", e.g., with the tissue surfaces defining the tract of the tissue "T." The introducer 100 will be described in greater detail hereinbelow.

With continued reference to FIG. 1, surgical portal 10 includes one or more longitudinal ports 12 extending generally parallel to the longitudinal axis "L" of the surgical portal 10. The longitudinal ports 12 are dimensioned to receive a surgical object, e.g. a surgical instrument (not shown) therethrough. Surgical portal 10 is adapted to be enclosed within introducer 100. Upon introduction of an instrument or surgical object (not shown) through a respective longitudinal port 12, the inner surface portions defining the longitudinal port 12 establish and maintain a substantial sealed relation about the instrument and/or surgical object. Surgical portal 10 may define an hourglass shape as shown. In this manner, trailing and leading ends 14, 16 may define flange segments, which may be integrally formed with surgical portal 10. Surgical portal 10 may be made from a disposable, compressible, and/or flexible type material, for example, but not limited to, a suitable foam, gel material, or soft rubber having sufficient flexibility to form a seal about one or more surgical objects, and also establish a sealing relation with tissue and/or the introducer 100. The foam may be sufficiently flexible to accommodate off axis motion of the surgical object. In one embodiment, the foam includes a polyisoprene material.

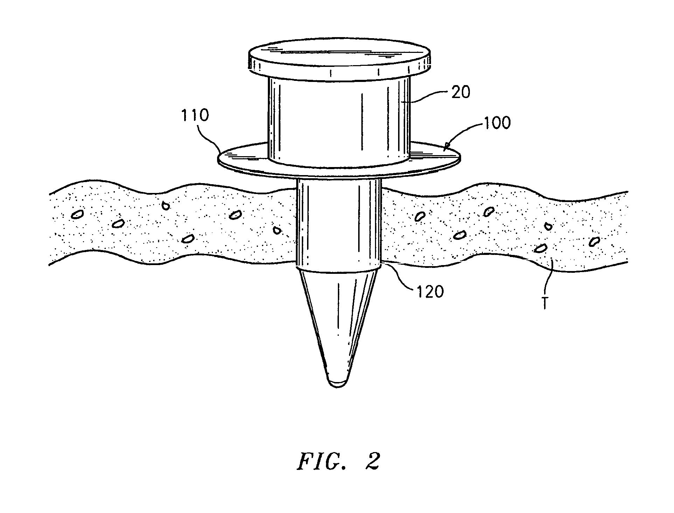

With reference to FIGS. 1 and 2, introducer 100 is adapted to facilitate insertion of surgical portal 10 within the tract defined within tissue "T." Introducer 100 is substantially elongated having a trailing or proximal end 102 and a leading or distal end 104 defining a longitudinal axis "Y." Introducer 100 includes a surface member 110 and a portal member 120 extending therefrom. Introducer 100 has a longitudinal channel 112 extending through surface member 110 and portal member 120 for reception and passage of surgical portal 10. Introducer 100 may be made of any type of suitable rigid material, for example, including but not limited to, metals and/or polymers.

Surgical portal 10 may be compressed to a compressed condition to permit at least partial passage through (insertion or extraction) the longitudinal channel 112 of the introducer 100. Once within the longitudinal channel 112, surgical portal 10 will return toward the normal expanded condition with an outer wall of the surgical portal 10 establishing a seal with the longitudinal channel 112. If the surgical portal 10 is then compressed so that the introducer 100 may be removed from the tract defined in tissue "T" (while leaving the surgical portal 10 in situ), the surgical portal 10 will also transition (after removing the applied compressive force) towards the expanded condition since the surgical portal 10 is naturally biased towards the expanded condition regardless of its position relative to the longitudinal channel 112. When in the expanded condition again, the surgical portal 10 establishes a seal with the tract defined in tissue "T." Surgical portal 10 may include an insufflation conduit 18 mounted within one of ports 12, or a separate port 18a (FIG. 1), and connectable to a source of insufflation fluid to permit passage of an insufflation fluid (e.g., CO₂), to create and/or maintain a working space in the pneumoperitoneum. The physician may insert various instruments through the surgical portal 10 and the introducer 100 for performing a procedure while maintaining a substantially sealed relationship with the surgical site.

In embodiments of the present disclosure, surgical portal 10 and introducer 100 may come preassembled with surgical portal 10 disposed within introducer 100. In the alternative, surgical portal 10 may be positioned within introducer 100 at the surgical site.

In operation, the leading or distal end 104 of introducer 100 is positioned within the tract defined within tissue "T" and the leading end 104 is advanced to a predetermined depth. As best depicted in FIG. 2, the introducer 100 may be inserted and advanced through the tract defined within tissue "T" via an obturator 20 positioned within the longitudinal channel 112 of the introducer 100. Thereafter, leading or distal end 16 of surgical portal 10 is positioned within proximal or trailing end 102 of introducer 100 (if not preassembled as hereinabove discussed). Upon insertion, surgical portal 10 compresses to fit within the inner boundary of longitudinal channel 112 of introducer 100. Surgical portal 10 is advanced relative to tissue tract "T" by either advancing the surgical portal 10 within introducer 100 or advancing the introducer 100 further into the tract defined within tissue "T." As the surgical portal 10 is advanced through the longitudinal channel 112 of introducer 100, the surgical portal 10 expands toward its normal expanded condition in sealed engagement with the introducer 100. While maintaining the surgical portal 10 within the tract defined within tissue "T" (e.g., by compression), the introducer 100 may be removed, thereby enabling the surgical portal 10 to establish a substantially sealed relationship with the tract defined within tissue "T" upon expansion into the expanded condition. The physician separates the surgical portal 10 from the introducer 100 by compressing the surgical portal 10 and extracting the introducer 100 by grasping the surface member 110 and pulling the portal member 120 out of the tract defined within tissue "T" while the surgical portal 10 remains disposed through tissue "T." The physician may then insert various instruments through the surgical port 10 while maintaining a substantially sealed relationship with the surgical site.

Alternatively, the physician may remove both the introducer 100 and the surgical portal 10 in one step by grasping the surface member 110 and extracting both the introducer 100 and the surgical portal 10 simultaneously. As such, the use of the introducer 100 limits tissue damage and facilitates the efficient insertion and removal of the surgical portal 10 into and/or from the tract defined within tissue "T."

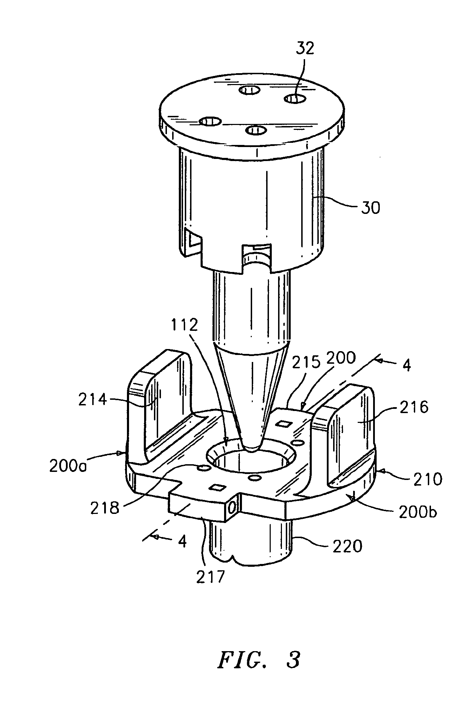

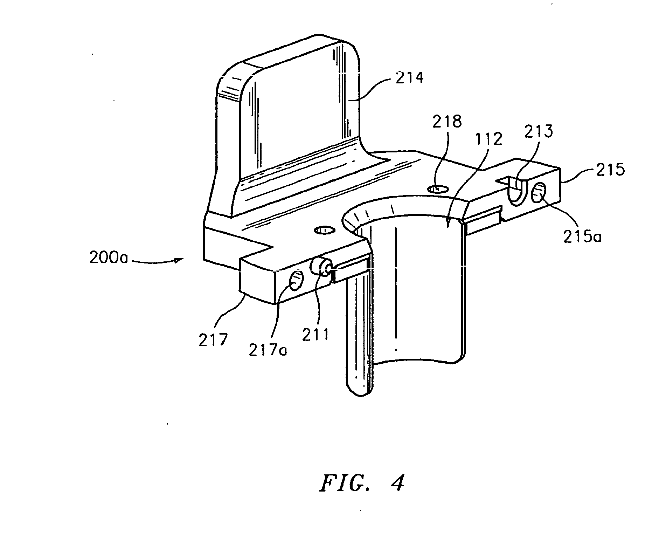

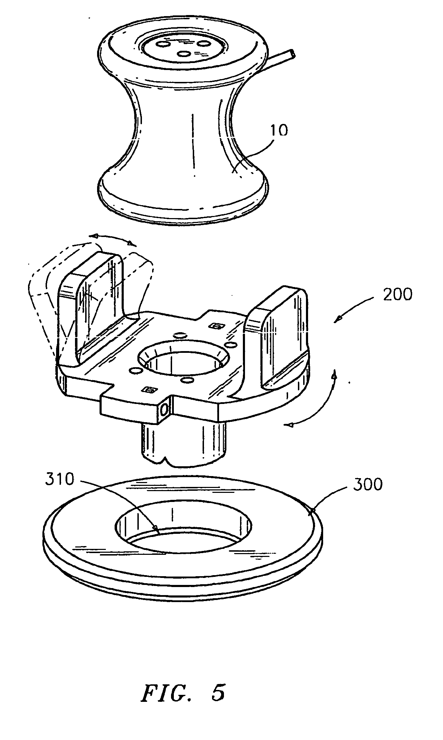

Another embodiment of an introducer 200 is illustrated in FIGS. 3-6. As best depicted in FIG. 4, introducer 200 is separable into first and second sections 200a, 200b to permit easier and more convenient removal thereof from a tract defined within tissue "T" (see FIGS. 1 and 2). In this embodiment, introducer 200 includes a surface member 210 and a portal member 220 extending therefrom. Surface member 210 includes first and second vertical projections 214, 216 and first and second horizontal projections 215, 217 extending therefrom. As illustrated in FIG. 5, vertical projections 214, 216 (see FIG. 3) are actuable between a first position and a second position in order to remove the introducer 200 and/or the surgical portal 10 and/or separating sections 200a, 200b (see FIG. 3). Surface member 210 further includes a plurality of longitudinal passages 218 extending therethrough which are disposed about longitudinal channel 112. Longitudinal passages 218 may be configured to longitudinally align with longitudinal openings 32 (e.g., for aligning the introducer 200 and the surgical port 10) defined in an obturator 30 that is insertable within longitudinal channel 112 for advancing introducer 200 into a tract defined within tissue "T." Referring again to FIG. 4, horizontal projections 215, 217 include horizontal bores 215a, 217a defined therethrough, aligning sections 200a, 200b (FIG. 3). Surface member 210 (FIG. 3) includes a pin 211 and groove 213 disposed on each section of the introducer 200. The pin 211 on first section 200a is configured to engage a groove, substantially similar to groove 213 on the second section 200b. Similarly, the groove 213 on the first section 200b is configured to engage a pin, substantially similar to pin 211 on the second section 200a, holding sections 200a, 200b together in releasable fashion.

Referring again to FIG. 5, another embodiment of a surgical portal and introducer assembly may include a spacer 300 defining a longitudinal aperture 310. The spacer 300 may be positioned between the tissue surface and introducer 100, 200.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-

A surgical portal and introducer assembly, which comprises:
an introducer dimensioned for at least partial positioning within a tissue tract and generally secured to the tissue tract in substantial sealed relationship with the tissue tract, the introducer having a surface member and a portal member extending from the surface member, the surface member being dimensioned to limit the positioning of the portal member within a tissue tract relative to an outer tissue surface, the portal member and the surface member defining a longitudinal channel therethrough; and
a portal positionable within the longitudinal channel of the introducer, the portal having at least one longitudinal port dimensioned to permit passage of a surgical object therethrough, the portal includes a compressible material that permits the portal to transition between a first expanded condition and a second compressed condition to facilitate passage through the longitudinal channel of the introducer, the portal being biased toward the first expanded condition, wherein the portal maintains a substantially sealed relationship with the longitudinal channel of the introducer when positioned in the longitudinal channel and disposed in the expanded condition.

The surgical portal and introducer assembly of paragraph [0032], further comprising a spacer that couples with the introducer.

The surgical portal and introducer assembly of paragraph [0032], wherein the introducer is separable into first and second sections.

The surgical portal and introducer assembly of paragraph [0034] wherein at least one of the first and second sections includes at least one groove and at least one of the first and second sections includes at least one pin extending therefrom, wherein the at least one pin and the at least one groove are engageable to couple the first and second sections.

The surgical portal and introducer assembly of paragraph [0032], wherein the surface member defines a plurality of passages therethrough, wherein at least one passage is dimensioned for the reception of a surgical object.

The surgical portal and introducer assembly of paragraph [0036], wherein at least one passage of the surface member is disposed in mechanical cooperation with an insufflation conduit for the reception of insufflation fluid.

The surgical portal and introducer assembly of paragraph [0032], wherein the surface member includes at least one projection extending therefrom, the at least one projection being actuable between a first position and at least one second position.

The surgical portal and introducer assembly of paragraph [0038] wherein in the first position, the at least one projection extends vertically from the surface member and is substantially parallel with a longitudinal axis of the introducer, and in the at least one second position, the at least one projection is disposed at an angle relative to the longitudinal axis of the introducer.

The surgical portal and introducer assembly of paragraph [0032], wherein the introducer is formed of a substantially rigid material and the portal is formed of a non-rigid material.

The surgical portal and introducer assembly of paragraph [0040], wherein the rigid material comprises at least one of steel or plastic.

The surgical portal and introducer assembly of paragraph [0040] wherein the non-rigid material comprises at least one of foam, gel material or soft rubber.

The surgical portal and introducer assembly of paragraph [0032], wherein the portal defines a plurality of longitudinal ports dimensioned to permit passage of a surgical object therethrough.

The surgical portal and introducer assembly of paragraph [0032], wherein the portal includes a flange segment on each of the leading and trailing ends such that the portal defines a substantially hour-glass shape.

The surgical portal and introducer assembly of paragraph [0044], wherein each of the flange segments has a larger diameter than the diameter of the longitudinal channel of the introducer when the portal is in the expanded condition.

The surgical portal and introducer assembly of paragraph [0032], wherein the portal is formed of a material having sufficient flexibility to accommodate off-axis motion of a surgical object positioned within the at least one longitudinal port.

The surgical portal and introducer assembly of paragraph [0032], wherein the at least one longitudinal port is substantially parallel to a longitudinal axis of the portal.

A method of accessing a surgical site, comprising the steps of:
providing an introducer having a surface member and a portal member extending from the surface member, wherein the surface member and the portal member define a longitudinal channel therethrough;
providing a portal having at least two longitudinal ports; and
advancing the introducer into tissue to a predetermined depth;
compressing the portal to fit within the longitudinal channel of the introducer;
advancing the portal at least partially through the longitudinal channel of the introducer; and
expanding the portal into sealing engagement with the longitudinal channel of the introducer.

The method of paragraph [0048], further comprising the step of simultaneously advancing at least two surgical objects through the at least two longitudinal ports of the portal.

The method of paragraph [0048], wherein the introducer includes at least one vertical projection extending from the surface member, the method further comprising the step of actuating the at least one vertical projection in order to remove the introducer from the tissue.

The method of paragraph [0048], further comprising the step of advancing the introducer into tissue with an obturator.

An introducer assembly according to any preceding claim having a surface member and a portal member extending from the surface member, wherein the surface member and the portal member define a longitudinal channel therethrough; for use in a method of accessing a surgical site, wherein the method comprises the step of:
providing an introducer assembly according to any preceding claim;
providing a portal having at least two longitudinal ports; and
advancing the introducer into tissue to a predetermined depth;
compressing the portal to fit within the longitudinal channel of the introducer;
advancing the portal at least partially through the longitudinal channel of the introducer; and
expanding the portal into sealing engagement with the longitudinal channel of the introducer.

The introducer assembly according to paragraph [0052], further comprising the step of simultaneously advancing at least two surgical objects through the at least two longitudinal ports of the portal.

The introducer assembly according to paragraph [0052] or paragraph [0053], wherein the introducer includes at least one vertical projection extending from the surface member, the method further comprising the step of actuating the at least one vertical projection in order to remove the introducer from the tissue.

The introducer assembly according to any of paragraphs [0052] to [0054], further comprising the step of advancing the introducer into tissue with an obturator.

## Claims

1. A surgical portal and introducer assembly, which comprises:
an introducer dimensioned for at least partial positioning within a tissue tract and generally secured to the tissue tract in substantial sealed relationship with the tissue tract, the introducer having a surface member and a portal member extending from the surface member, the surface member being dimensioned to limit the positioning of the portal member within a tissue tract relative to an outer tissue surface, the portal member and the surface member defining a longitudinal channel therethrough; and
a portal positionable within the longitudinal channel of the introducer, the portal having at least one longitudinal port dimensioned to permit passage of a surgical object therethrough, the portal includes a compressible material that permits the portal to transition between a first expanded condition and a second compressed condition to facilitate passage through the longitudinal channel of the introducer, the portal being biased toward the first expanded condition, wherein the portal maintains a substantially sealed relationship with the longitudinal channel of the introducer when positioned in the longitudinal channel and disposed in the expanded condition.

2. The surgical portal and introducer assembly of claim 1, further comprising a spacer that couples with the introducer, preferably wherein the introducer is separable into first and second sections.

3. The surgical portal and introducer assembly of claim 2, wherein at least one of the first and second sections includes at least one groove and at least one of the first and second sections includes at least one pin extending therefrom, wherein the at least one pin and the at least one groove are engageable to couple the first and second sections.

4. The surgical portal and introducer assembly of any preceding claim, wherein the surface member defines a plurality of passages therethrough, wherein at least one passage is dimensioned for the reception of a surgical object; preferably wherein at least one passage of the surface member is disposed in mechanical cooperation with an insufflation conduit for the reception of insufflation fluid.

5. The surgical portal and introducer assembly of any preceding claim, wherein the surface member includes at least one projection extending therefrom, the at least one projection being actuable between a first position and at least one second position.

6. The surgical portal and introducer assembly of claim 5, wherein in the first position, the at least one projection extends vertically from the surface member and is substantially parallel with a longitudinal axis of the introducer, and in the at least one second position, the at least one projection is disposed at an angle relative to the longitudinal axis of the introducer.

7. The surgical portal and introducer assembly of any preceding claim, wherein the introducer is formed of a substantially rigid material and the portal is formed of a non-rigid material.

8. The surgical portal and introducer assembly of claim 7, wherein the rigid material comprises at least one of steel or plastic; and/or wherein the non-rigid material comprises at least one of foam, gel material or soft rubber.

9. The surgical portal and introducer assembly of any preceding claim, wherein the portal defines a plurality of longitudinal ports dimensioned to permit passage of a surgical object therethrough.

10. The surgical portal and introducer assembly of any preceding claim, wherein the portal includes a flange segment on each of the leading and trailing ends such that the portal defines a substantially hour-glass shape; preferably wherein each of the flange segments has a larger diameter than the diameter of the longitudinal channel of the introducer when the portal is in the expanded condition.

11. The surgical portal and introducer assembly of any preceding claim, wherein the portal is formed of a material having sufficient flexibility to accommodate off-axis motion of a surgical object positioned within the at least one longitudinal port.

12. The surgical portal and introducer assembly of any preceding claim, wherein the at least one longitudinal port is substantially parallel to a longitudinal axis of the portal.

13. An introducer assembly according to any preceding claim having a surface member and a portal member extending from the surface member, wherein the surface member and the portal member define a longitudinal channel therethrough; for use in a method of accessing a surgical site, wherein the method comprises the step of:
providing an introducer assembly according to any preceding claim;
providing a portal having at least two longitudinal ports; and
advancing the introducer into tissue to a predetermined depth;
compressing the portal to fit within the longitudinal channel of the introducer;
advancing the portal at least partially through the longitudinal channel of the introducer; and
expanding the portal into sealing engagement with the longitudinal channel of the introducer.

14. The introducer assembly according to claim 13, further comprising the step of simultaneously advancing at least two surgical objects through the at least two longitudinal ports of the portal.

15. The introducer assembly according to claim 13 or claim 14, wherein the introducer includes at least one vertical projection extending from the surface member, the method further comprising the step of actuating the at least one vertical projection in order to remove the introducer from the tissue.

16. The introducer assembly according to any of claims 13 to 15, further comprising the step of advancing the introducer into tissue with an obturator.
